# EUROPEAN PATENT SPECIFICATION

(11) **EP 3 838 253 B1**
(45) Date of publication and mention of the grant of the patent: **13.03.2024**
(21) Application number: 18935713.0
(22) Date of filing: 27.09.2018
(51) Int. Cl.: A61K 8/73, A61K 8/02, A61K 8/34, A61Q 19/00, B65D 83/68, A61K 8/04

(54) **GELLED AEROSOL PRODUCT AND GEL FORMING KIT**
GELIERTES AEROSOLPRODUKT UND GELBILDENDES KIT
PRODUIT AÉROSOL GÉLIFIÉ ET KIT DE FORMATION DE GEL

(43) Date of publication of application: 23.06.2021
(73) Proprietor: Toyo Aerosol Industry Co., Ltd., Tokyo 141-0022 (JP)
(72) Inventor: SEKIYA, Keiji, Toyonaka-shi, Osaka 560-0083 (JP); NISHIO, Tomoyuki, Toyonaka-shi, Osaka 560-0083 (JP); KUWANA, Yuki, Toyonaka-shi, Osaka 560-0083 (JP); NISHIKATA, Yuri, Tokyo 141-0022 (JP); TERASHIMA, Remi, Tokyo 141-0022 (JP); NAKAJIMA, Yasutomo, Tokyo 141-0022 (JP); KAMIJYO, Hokuto, Tokyo 141-0022 (JP)
(74) Representative: Vigand, Philippe
(86) International application number: PCT/JP2018/036027
(87) International publication number: WO 2020/065849

(56) References cited:
- WO-A1-2007/105526
- WO-A1-2012/165309
- JP-A- H08 283 305
- JP-A- 2006 069 952
- JP-A- 2008 050 298
- JP-A- 2008 050 298
- JP-A- 2014 047 179
- JP-A- 2014 181 192
- JP-A- 2016 169 186
- JP-A- 2016 190 874
- KR-B1- 101 731 926
- US-A1- 2009 130 039
- US-A1- 2014 005 615

## Description

### [Technical Field]

The present invention relates to a gelled aerosol product and a gel forming kit, which can be used for peel-off mask cosmetics and the like.

### [Background Art]

Conventionally, peel-off mask cosmetics such as peel-off masks are mainly in the form of a sheet obtained by impregnating a nonwoven fabric with a skin toner component; however, a defined sheet shape makes it difficult to use such a sheet on an arbitrary spot. There are also cream-form and clay-form peel-off mask cosmetics that can be used on any spot; however, these cosmetics need to be washed off and thus cannot sufficiently leave their beauty components on the skin.

To address these problems, gel-form peel-off mask cosmetics which can be used on any spot and removed by peeling after use have been developed. For example, Non-patent Document 1 discloses a two-component peel-off mask that utilizes gelation induced by mixing tamarind gum and glycerin.

### [Citation List]

### [Non-patent Document]

[Non-patent Document 1] Tomoyuki Nishio and Sanae Hojo, "Physical Properties of Natural Polymers and its Application for Cosmetics, p. 71, 5-2. Two-Component Carbonic Acid Peel-off Mask, Table 3", FRAGRANCE JOURNAL, October 2014

### [Patent Documents]

KR 101 731 926 B1 relates to a pouch assembly. WO 2012/165309 A1 discloses a composition for external use. JP 2008-050298 A relates to an external gel composition for attachment to skin.

### [Summary of Invention]

### [Technical Problem]

However, the peel-off mask disclosed in the above-described document has a problem in that the gelation takes a long time of 15 to 20 minutes. Therefore, the present inventors investigated the use of a diol having a small number of carbon atoms, such as butylene glycol, and glycerin as components that allow the gelation to proceed by reacting with tamarind gum, in place of glycerin and sorbitol that are used in the above-described document. As a result, it was found that, although such use accelerated the gelation, the resulting gel was soft and syneresis occurred over time, causing a problem that, for example, the gel is peeled off during its use as a peel-off mask.

The present invention is aimed at solving the above-described problems. That is, an object of the present invention is to provide: a gelled aerosol product which can be quickly gelled and form a good film in which syneresis is inhibited; and a gel forming kit.

### [Solution to Problem]

The present invention relates to a gelled aerosol product which includes: a first container filled with a first liquid; a second container filled with a second liquid; and a propellant, and having a discharge mechanism for discharging the first liquid and the second liquid, the gelled aerosol product being characterized in that
the first liquid contains either one of the following (A) and (B), and tamarind gum,
the second liquid contains the other of the following (A) and (B), glycerin, and a diol having 2 to 6 carbon atoms:
   (A) at least one selected from the group consisting of locust bean gum, glucomannan, tara gum, and guar gum; and
   (B) xanthan gum, and
the first liquid and the second liquid that are discharged are gelled by mixing.

### [Advantageous Effects of Invention]

According to the present invention, a gelled aerosol product which can be quickly gelled and form a good film in which syneresis is inhibited, and a gel forming kit can be provided.

### [Brief Description of the Drawings]

[FIG. 1] FIG. 1 is a schematic drawing that illustrates one example of an aerosol product of a double-structure container.
[FIG. 2] FIG. 2 is a schematic drawing that illustrates a double can type aerosol product.

### [Description of Embodiments]

In the present invention, unless otherwise specified, the descriptions indicating numerical ranges, such as "from XX to YY" and "XX to YY", each mean a numerical range that includes its lower limit value and upper limit value as end points.

The gelled aerosol product of the present invention contains, in addition to tamarind gum contained in the first liquid, and glycerin and the diol having 2 to 6 carbon atoms that are contained in the second liquid:
(A) at least one selected from the group consisting of locust bean gum, glucomannan, tara gum, and guar gum (hereinafter, may be simply referred to as "(A)"); and
(B) xanthan gum (hereinafter, may be simply referred to as "(B)").

The (A) and the (B) may be contained in either one of the first liquid and the second liquid. A mode in which the first liquid contains the (A) and the second liquid contains the (B), or a mode in which the first liquid contains the (B) and the second liquid contains the (A) may be adopted. Preferably, the first liquid contains the (A) while the second liquid contains (B).

The (A) and the (B) react with each other to form a gel. In addition to the gel formation by tamarind gum, glycerin and the diol having 2 to 6 carbon atoms, the gel formation by the (A) and the (B) complements the gel formation of tamarind gum, allowing the formation of a good film in which syneresis is inhibited. Although the components of (A) and (B) are normally heat-melted and gelled, the above-described effects are obtained since the components of (A) and (B) form a soft and elastic gel even when they are mixed at normal temperature (e.g., about 20 to 35°C).

From the standpoint of complementing the gel formation, as a combination of components used for the (A) and the (B), it is possible to employ, for example, a combination of locust bean gum and carrageenan, a combination of a calcium salt of phosphoric acid, hydrochloric acid, lactic acid or the like and sodium alginate, a combination of a potassium salt or sodium salt of phosphoric acid, hydrochloric acid, lactic acid or the like and carrageenan, or a combination of a calcium salt of phosphoric acid, hydrochloric acid, lactic acid or the like and gellan gum; however, from the standpoint of attaining favorable gel formation, the above-described specific combination of the (A) and the (B) is appropriate.

From the standpoint of inhibiting syneresis to obtain a gel having a good strength, the (A) is preferably at least one selected from the group consisting of locust bean gum, tara gum, and guar gum, more preferably locust bean gum.

The ratio of the (A) and the (B), [(A):(B)], is preferably 1:3 to 3:1, more preferably 1:2 to 2:1, still more preferably 2:3 to 3:2, in terms of mass.

The content of the (A) in the first liquid or the second liquid is preferably 0.05% by mass to 5.0% by mass, more preferably 0.1% by mass to 2.0% by mass, still more preferably 0.15% by mass to 1.0% by mass.

The content of the (B) in the first liquid or the second liquid is preferably 0.05% by mass to 5.0% by mass, more preferably 0.1% by mass to 2.0% by mass, still more preferably 0.15% by mass to 1.0% by mass.

The first liquid contains tamarind gum. Tamarind gum reacts with glycerin and the diol having 2 to 6 carbon atoms to form a network based on hydrogen bonds and is thereby gelled. An elastic gel having good feeling of use is formed by using tamarind gum, and glycerin and the diol having 2 to 6 carbon atoms provide moisture to the skin as moisturizing components. In addition, it is not necessary to wash off the first liquid when it is used as, for example, a peel-off mask cosmetic since it can be cleanly removed after gelation.

Further, tamarind gum is unlikely to undergo a change in viscosity even when it is used in an aerosol product; therefore, tamarind gum can be used in a stable manner. This is believed to be because tamarind gum exhibits substantially constant Newtonian viscosity even when a shear force is applied thereto.

The tamarind gum used in the present invention is not particularly restricted as long as it is a polysaccharide (xyloglucan) obtained from tamarind seeds, and may be a commercially available product. Examples thereof include GLYLOID 6C, GLYLOID 3S, and GLYLOID 2A (which are manufactured by DSP Gokyo Food & Chemical Co., Ltd.), among which GLYLOID 6C having excellent transparency is preferred.

The content of tamarind gum in the first liquid is preferably 1.0% by mass to 10.0% by mass, more preferably 1.5% by mass to 8.0% by mass, still more preferably 2.0% by mass to 5.0% by mass, yet still more preferably 2.5% by mass to 4.5% by mass, particularly preferably 3.0% by mass to 4.0% by mass.

In the present invention, the second liquid is required to contain glycerin and a diol having 2 to 6 carbon atoms. This relationship can increase the gelling rate.

From the standpoint of the gelling rate, the mass ratio of the diol having 2 to 6 carbon atoms with respect to glycerin (diol having 2 to 6 carbon atoms/glycerin) is preferably 0.15 to 7.0, more preferably 0.3 to 5.0, still more preferably 0.4 to 4.0, yet still more preferably 0.4 to 3.0, particularly preferably 1.0 to 2.0.

In the second liquid, the content of the diol having 2 to 6 carbon atoms is preferably 5.0% by mass to 70.0% by mass, more preferably 10.0% by mass to 65.0% by mass, still more preferably 15.0% by mass to 55.0% by mass, particularly preferably 25.0% by mass to 45.0% by mass. When the content of the diol having 2 to 6 carbon atoms is in this range, gelation occurs quickly, and a gel having a good elasticity can be obtained.

In the second liquid, the content of glycerin is preferably 10.0% by mass to 70.0% by mass, more preferably 15.0% by mass to 60.0% by mass, still more preferably 25.0% by mass to 55.0% by mass, particularly preferably 25.0% by mass to 45.0% by mass. When the content of glycerin is in this range, a preferable elastic gel is likely to be formed by glycerin and tamarind gum.

The diol having 2 to 6 carbon atoms is preferably a diol having 3 to 5 carbon atoms. Specific examples thereof include propylene glycol, 1,3-propanediol, 1,2-butylene glycol, 1,3-butylene glycol, 1,4-butylene glycol, 2,3-butylene glycol, isopentyl diol, neopentyl glycol, pentylene glycol, and hexylene glycol.

The diol having 2 to 6 carbon atoms is preferably at least one selected from the group consisting of 1,3-butylene glycol, propylene glycol, isopentyl diol, pentylene glycol and 1,3-propanediol, more preferably 1,3-butylene glycol.

In the present invention, a thickening agent may be further used in addition to the above-described tamarind gum, (A) and (B). The thickening agent is not particularly restricted, and any known thickening agent can be used. Examples thereof include carboxyvinyl polymers, acrylic acid-alkyl acrylate copolymers, acrylates/alkyl acrylate crosspolymers, Na polyacrylates, cellulose gums, (PEG-240/decyltetradeceth-20/HDI) copolymers, and polyurethanes. The thickening agent is preferably a carboxyvinyl polymer.

By using such a thickening agent, the viscosity and the fluidity can be appropriately controlled, so that, for example, the equal-amount dischargeability and the like are improved when the thickening agent is used in an aerosol product. From the standpoint of the size of an actuator (discharge mechanism), the thickening agent is likely to favorably control the viscosity and the fluidity even in an aerosol product of a double-structure container in which the freedom in the design, such as the diameter of an actuator passage, is low and a fine adjustment of the discharge amount is difficult.

The thickening agent may be used in either one or both of the first liquid and the second liquid. The content of the thickening agent other than the above-described tamarind gum, (A) and (B) in the first liquid or the second liquid is preferably 0.05% by mass to 2.0% by mass, more preferably 0.1% by mass to 1.0% by mass.

The viscosity of the first liquid is preferably 1,000 mPa•s to 100,000 mPa•s, more preferably 5,000 mPa•s to 50,000 mPa•s, still more preferably 10,000 mPa•s to 40,000 mPa•s.

The viscosity of the second liquid is preferably 1,000 mPa•s to 100,000 mPa•s, more preferably 2,000 mPa•s to 80,000 mPa•s, still more preferably 5,000 mPa•s to 70,000 mPa•s, yet still more preferably 7,000 mPa•s to 60,000 mPa•s. When the viscosity is in the above-described respective ranges, dripping of each liquid can be inhibited at the time of mixing. In addition, favorable discharge properties are attained in the aerosol product.

The viscosity is measured using a B-type viscometer under the conditions of 25°C and 6 rpm.

The pH of the first liquid is not particularly restricted as long as it is in the range of an ordinary cosmetic, and can be widely set in an acidic to alkaline range; however, in a peel-off mask cosmetic, the pH of the first liquid is preferably weakly acidic to weakly alkaline, i.e. 4.0 to 9.0, more preferably 5.0 to 8.0.

The pH of the second liquid is not particularly restricted as long as it is in the range of an ordinary cosmetic, and can be widely set in an acidic to alkaline range; however, in a peel-off mask cosmetic, the pH of the second liquid is preferably weakly acidic to weakly alkaline, i.e. 4.0 to 9.0, more preferably 5.0 to 8.0.

For pH adjustment, a known pH modifier can be used. Examples thereof include potassium hydroxide, citric acid, and sodium citrate.

### <Other Additives>

In the first liquid and the second liquid, other additives may also be used to such an extent that does not impair the effects of the present invention. For example, known surfactant, thickening agent, and fragrance can be added. In addition, optional active ingredients, such as a beauty component in the case of using the liquids as a peel-off mask, can be added as well.

These other additives may be added to either the first liquid or the second liquid.

The form of the gel forming kit is not particularly restricted, and examples thereof include a kit which includes a first container containing the first liquid and a second container containing the second liquid. The first liquid and the second liquid can be gelled by mixing.

The form of the gel forming kit is not particularly restricted as long as it is a container in which the first liquid and the second liquid can be separately filled and stored and from which these liquids can be taken out as appropriate, and examples of such a container include a bottle container, a pump container, a cup container, ajar container, a tube container, a pouch container, a pack container, and a bag.

The mixing ratio of the first liquid and the second liquid is not particularly restricted since it varies depending on the concentrations of the raw materials used in the first liquid and the second liquid; however, the mixing ratio of the first liquid and the second liquid (mass of first liquid:mass of second liquid) is preferably 1.0:5.0 to 5.0:1.0, more preferably 1.0:2.0 to 2.0:1.0. When the mixing ratio is in this range, the first liquid and the second liquid can be easily mixed.

In the case of a gelled aerosol product, the mixing ratio of the first liquid and the second liquid that are discharged from an actuator (discharge mechanism) (mass of first liquid:mass of second liquid) is not particularly restricted because it varies depending on the concentrations of the respective raw materials used in the first liquid and the second liquid; however, it is preferably 0.8:1.2 to 1.2:0.8, more preferably 0.9:1.1 to 1.1:0.9.

The first liquid and the second liquid that are discharged can be used by, for example, mixing them with a finger or the like on the palm of a hand or a tray at the above-described mixing ratio. After the mixing, the resulting mixture can be applied to an arbitrary spot and gelled.

Preferred contents of the respective raw materials in the gel forming kit and the gelled aerosol product at the start of mixing the first liquid and the second liquid are described below. It is preferred to adjust the contents as described below by adjusting the above-described mixing ratio as appropriate.

The content of the (A) at the time of mixing is preferably 0.02% by mass to 2.5% by mass, more preferably 0.05% by mass to 1.0% by mass, still more preferably 0.07% by mass to 0.5% by mass.

The content of the (B) at the time of mixing is preferably 0.02% by mass to 2.5% by mass, more preferably 0.05% by mass to 1.0% by mass, still more preferably 0.07% by mass to 0.5% by mass.

The content of tamarind gum at the time of mixing is preferably 0.5% by mass to 5.0% by mass, more preferably 0.7% by mass to 4.0% by mass, still more preferably 1.0% by mass to 2.5% by mass, yet still more preferably 1.25% by mass to 2.25% by mass, particularly preferably 1.5% by mass to 2.0% by mass.

The content of the diol having 2 to 6 carbon atoms at the time of mixing is preferably 2.5% by mass to 35.0% by mass, more preferably 5.0% by mass to 32.5% by mass, still more preferably 7.5% by mass to 27.5% by mass, particularly preferably 12.5% by mass to 22.5% by mass.

The content of glycerin at the time of mixing is preferably 5.0% by mass to 35.0% by mass, more preferably 7.5% by mass to 30.0% by mass, still more preferably 12.5% by mass to 27.5% by mass, particularly preferably 12.5% by mass to 22.5% by mass.

### <Aerosol Product>

The gelled aerosol product includes: a first container filled with the first liquid; and a second container filled with the second liquid. The gelled aerosol product may take any form of a container, such as a double-structure container or a double can type container, as long as it can be filled with the first liquid and the second liquid separately and discharge both of these liquids.

The double-structure container includes, for example, an outer container including: a propellant filling space filled with a propellant; the first container; and the second container, and has a discharge mechanism for discharging the first liquid and the second liquid from the first container and the second container, respectively, and the propellant filling space is formed between the first and the second containers and the outer container.

The double-structure container has an outer container including: the propellant filling space filled with a propellant; the first container filled with the first liquid; and the second container filled with the second liquid. By the pressure of the propellant filled into the propellant filling space, the first liquid and the second liquid can be discharged simultaneously from the first container and the second container, respectively.

Examples of a double-structure container that includes a discharge mechanism will now be described referring to the drawings.

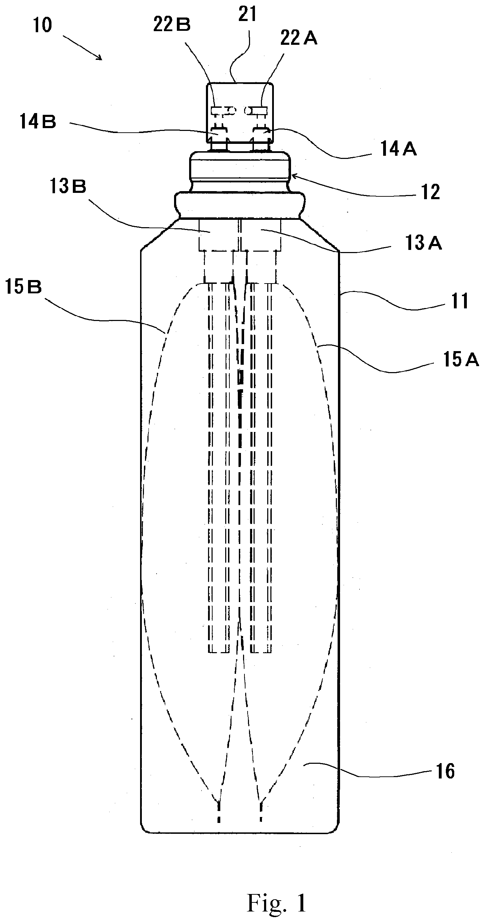

FIG. 1 is a schematic drawing that illustrates one example of an aerosol product having a double-structure container.

This aerosol product 10 includes an outer container 11 made of a metal, on which an aerosol valve 12 is provided. Inside of this outer container 11, a first inner bag 15A which partitions the first container to be filled with the first liquid, and a second inner bag 15B which partitions the second container to be filled with the second liquid are provided. In addition, a propellant filling space 16 to be filled with a propellant is formed by the gaps between the outer container 11, the first inner bag 15A, and the second inner bag 15B.

On the aerosol valve 12, a first stem 14A and a second stem 14B, which are arranged in a vertically slidable manner in a first housing 13A and a second housing 13B, respectively, and each have a stem passage inside, are provided. On the upper ends of these first stem 14Aand second stem 14B, a common actuator (discharge mechanism) 21 is provided.

In FIG. 1, those constituents positioned inside the outer container 11 and the actuator 21 are illustrated with dashed lines.

In the common actuator 21, a first actuator passage 22A which is in communication with the stem passage of the first stem 14A, and a second actuator passage 22B which is in communication with the stem passage of the second stem 14B are provided. The actuator passages 22A and 22B are each in communication with a discharge port such that the first liquid and the second liquid can be discharged therefrom.

The inner diameters of the actuator passages 22A and 22B and discharge ports may be changed as appropriate in accordance with the viscosity of the liquid to be discharged. The first liquid and the second liquid that are discharged from the respective discharge ports can be gelled by mixing with a finger or the like on, for example, the palm of a hand or a tray.

In FIG. 1, a mode of discharging two kinds of liquid contents, which are the first liquid and the second liquid, is illustrated; however, the double-structure container may be in a mode of discharging three or more kinds of liquid contents. In other words, by further providing a third inner bag, a third housing, a third stem and the like in the outer container 11, three or more kinds of liquid contents can be discharged. For example, the third inner bag and a fourth inner bag may be provided so that the above-described other additives, active ingredients and the like can be filled thereinto and discharged therefrom.

Further, the inner bags are parallelly arranged in FIG. 1; however, various known modes can be adopted for the arrangement of the inner bags. The first inner bag and the second inner bag may be arranged one above the other. For example, the first inner bag may be arranged above the second inner bag, and the first inner bag and the second inner bag may each be connected to the actuator via a tube or the like. The tube connecting the second inner bag and the actuator may be on the outside of the first inner bag, or may pass through the inside of the first inner bag in such a manner that the tube does not come into contact with the first liquid.

Moreover, in FIG. 1, a mode of discharging two kinds of liquid contents, which are the first liquid and the second liquid, from the two respective stems is illustrated; however, a mode of discharging the two kinds of liquid contents from a single stem having two flow paths inside may be adopted as well.

The propellant is not particularly restricted, and a liquefied gas or a compressed gas may be used. Examples of the liquefied gas include propane, butane, pentane, and a liquefied petroleum gas containing these gases, as well as dimethyl ether, hydrofluoroolefin, and hydrofluorocarbon. Examples of the compressed gas include nitrous oxide gas, nitrogen gas, carbon dioxide gas, and a mixed gas thereof. The propellant is preferably a compressed gas.

Meanwhile, the double can type container is a mode of simultaneously discharging the first liquid and the second liquid using a propellant from each of two aerosol products that are integrated.

For example, the double can type container includes: a first inner container filled with the first liquid; a second inner container filled with the second liquid; a first outer container in which the first inner container is housed; and a second outer container in which the second inner container is housed, and has a discharge mechanism for discharging the first liquid and the second liquid.

In this double can type container, the propellant is filled into each of a space formed between the first inner container and the first outer container and a space formed between the second inner container and the second outer container.

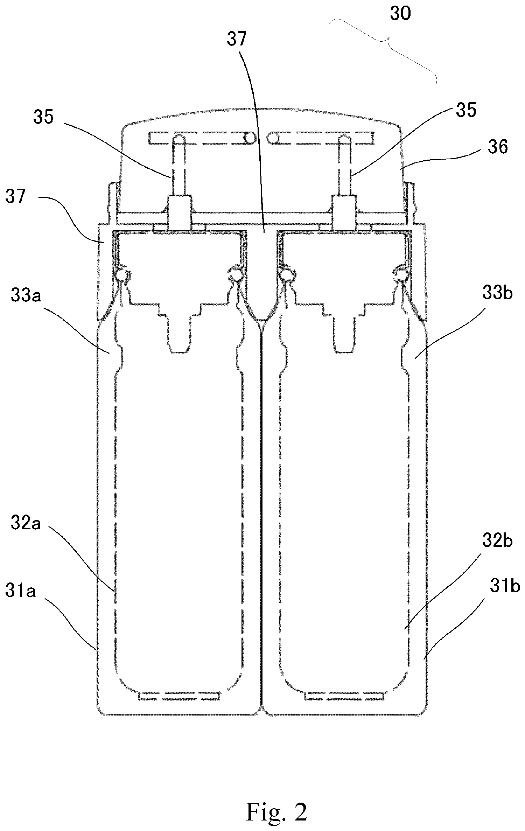

FIG. 2 is a schematic drawing that illustrates a double can type aerosol product.

An aerosol product 30 includes: a first outer container 31a, a second outer container 31b, and an actuator (discharge mechanism) 36 which includes actuator passages 35. The first outer container 31a houses a first inner container 32a, and this first inner container 32a is filled with the first liquid. The second outer container 3 1b houses a second inner container 32b, and this second inner container 32b is filled with the second liquid. The first outer container 31a and the second outer container 31b are fixed together by a fixation plate 37.

In this aerosol product, a first propellant filling space 33a and a second propellant filling space 33b are formed between the first outer container 31a and the first inner container 32a and between the second outer container 31b and the second inner container 32b, respectively, and these propellant filling spaces are each filled with a propellant for discharging the first liquid and the second liquid from the discharge mechanism 35.

In FIG. 2, the containers are parallelly arranged; however, various known modes can be adopted for the arrangement of the containers. For example, the first outer container and the second outer container may be arranged one above the other. The first outer container may be arranged above the second outer container, and the first outer container and the second outer container may each be connected to the actuator via a tube or the like.

Further, in FIG. 2, a double can type aerosol having inner containers is illustrated; however, at least one of the first outer container and the second outer container may be in a mode in which a liquid content and a propellant are filled into the same space without an inner container.

For example, the double can type aerosol may take a mode of having the first container filled with a propellant and the first liquid, the second container filled with a propellant and the second liquid, and a discharge mechanism for discharging the first liquid and the second liquid.

The discharge mechanism 35 has a discharge port from which compositions filled into the first inner container 32a and the second inner container 32b are discharged.

In the same manner as the above-described double-structure container, the double can type aerosol product may also be in a mode of discharging three or more kinds of liquid contents. In other words, by further providing a third outer container, a third inner container, a third propellant filling space and the like, three or more kinds of liquid contents can be discharged.

### <Container>

The containers (inner containers) to be filled with the first liquid and the second liquid are not particularly restricted. Known resin containers, metal containers, or a combination thereof can be used.

The outer containers are also not particularly restricted as long as they can withstand the pressure of a propellant, and known resin containers and metal containers can be used.

### EXAMPLES

The present invention will now be described concretely by way of Examples thereof. However, the present invention is not restricted to the modes of the following Examples.

### <Example 1: Production of Aerosol Product 1>

### (Preparation of First Liquid)

A first liquid was formulated as shown in Table 1. A dispersion was prepared by adding tamarind gum, locust bean gum and methylparaben to 1,3-butylene glycol (BG). This dispersion was added to water with stirring the water, and the resultant was heated to 80°C in a hot bath and then further stirred for 30 minutes to obtain a homogeneous paste. The thus obtained paste was adjusted to have a prescribed pH by adding thereto a 10% aqueous citric acid solution and a 10% aqueous sodium citrate solution, and this paste was subsequently cooled, whereby the first liquid was obtained.

### (Preparation of Second Liquid)

A second liquid was formulated as shown in Table 1. A dispersion was prepared by adding xanthan gum and methylparaben to a portion of BG. This dispersion and a carboxyvinyl polymer (hereinafter, referred to as "carbomer"; 2% aqueous solution) were added to water with stirring the water, and the resultant was stirred for 30 minutes to obtain a homogeneous paste. This paste was stirred with addition of glycerin and the remaining BG and, once the paste was in a homogeneous state, a 10% aqueous potassium hydroxide solution was added thereto so as to adjust the paste to have a prescribed pH, whereby the second liquid was obtained.

An aerosol container having a double structure including two independent first and second containers inside was prepared, and the above-prepared first liquid and second liquid were filled into the first container and the second container, respectively. In the aerosol container, a void formed between the first container and the second container was filled with nitrogen gas, whereby an aerosol product 1 was obtained. For the thus obtained first liquid, second liquid and aerosol product 1, the following evaluations were conducted. The results thereof are shown in Table 1.

### (Viscosity Measurement)

The first liquid and the second liquid were each placed in a beaker for viscosity measurement, and the beaker was left to stand in a 25°C thermostatic chamber for 2 hours, after which the viscosity was measured using a B-type viscometer at 6 rpm.

### (pH Measurement)

The electrode of a pH meter was immersed in each of the first liquid and the second liquid for at least 2 minutes, and the pH was read thereafter.

### (Evaluation of Gel)

The first liquid (5 g) and the second liquid (5 g) were mixed on the palm of a hand for 1 minute. Subsequently, the resulting mixture was applied to a forearm, and the state of the applied mixture was observed. The following five items were evaluated.

Gelling rate: The time until a gel state was maintained when the mixture was lifted after being left to stand was evaluated based on the following criteria.
4 points: less than 5 minutes
3 points: 5 minutes or more but less than 15 minutes
2 points: 15 minutes or more but less than 30 minutes
1 point: 30 minutes or more

Dripping: When the mixture was placed on the back of a hand and the palm of the hand was brought perpendicular to a desk, the dripping state of the mixture was visually observed and evaluated based on the following criteria.
4 points: No dripping was observed.
3 points: A slight dripping was observed.
2 points: A portion of the mixture dripped from the hand.
1 point: Most of the mixture dripped from the hand.

Film strength: When the mixture in a gel state was pressed with a finger, the hardness was evaluated based on the following criteria.
4 points: The gel did not collapse at all when pressed with a finger.
3 points: The gel hardly collapsed when pressed with a finger.
2 points: The gel collapsed when pressed with a finger.
1 point: No gelation occurred.

Syneresis of gel: The surface of the mixture in a gel state as well as the presence or absence of syneresis were visually observed and evaluated based on the following criteria.
4 points: No syneresis was observed.
3 points: Hardly any syneresis was observed.
2 points: Syneresis was observed.
1 point: Extremely severe syneresis was observed.

Feeling of use as peel-off mask: Assuming the use as a peel-off mask, the feeling of use in a series of operation from mixing of the first liquid and the second liquid to peeling of the resulting gel was evaluated based on the following criteria.
4 points: The feeling of use as a peel-off mask was very good.
3 points: The feeling of use as a peel-off mask was good.
2 points: The feeling of use as a peel-off mask was poor.
1 point: The feeling of use as a peel-off mask was very poor.

The evaluation items were each assessed as follows based on the average value of the above-described evaluation scores assigned by five panelists. The results thereof are shown in Table 1.
A: The average value of the evaluation scores was 3.5 or higher.
B: The average value of the evaluation scores was 3.0 or higher but lower than 3.5.
C: The average value of the evaluation scores was 2.5 or higher but lower than 3.0.
D: The average value of the evaluation scores was lower than 2.5.

### <Examples 2 to 13>

Aerosol products were obtained in the same manner as in Example 1, except that the formulations of the first liquid and the second liquid were changed as shown in Table 1. The evaluation results are shown in Table 1.

### <Comparative Examples 1 to 4>

Aerosol products were obtained in the same manner as in Example 1, except that the formulations of the first liquid and the second liquid were changed as shown in Table 2. The evaluation results are shown in Table 2.

**[Table 1]**

| | Example | | Example | | Example | | Example | | Example | | Example | | Example | |
|---|---|---|---|---|---|---|---|---|---|---|---|---|---|---|
| | 1 | | 2 | | 3 | | 4 | | 5 | | 6 | | 7 | |
| | FL | SL | FL | SL | FL | SL | FL | SL | FL | SL | FL | SL | FL | SL |
| Purified water | 89.1 | 11.3 | 89.3 | 11.3 | 89.9 | 12.0 | 89.9 | 17.5 | 89.9 | 17.5 | 89.9 | 17.5 | 89.9 | 17.5 |
| BG | 5.0 | 41.9 | 5.0 | 41.9 | 5.0 | 41.9 | 5.0 | 41.9 | 5.0 | 41.9 | 5.0 | 41.9 | 5.0 | 41.9 |
| Glycerin | - | 30.0 | - | 30.0 | - | 30.0 | - | 30.0 | - | 30.0 | - | 30.0 | - | 30.0 |
| Tamarind gum | 4.3 | - | 4.0 | - | 3.5 | - | 3.5 | - | 3.5 | - | 3.5 | - | 3.5 | - |
| Locust bean gum | 0.2 | - | 0.3 | - | 0.2 | - | 0.2 | - | - | 0.2 | - | - | - | - |
| Xanthan gum | - | 0.2 | - | 0.2 | - | 0.5 | - | 0.2 | 0.2 | - | - | 0.2 | - | 0.2 |
| Guar gum | - | - | - | - | - | - | - | - | - | - | 0.2 | - | - | - |
| Tara gum | - | - | - | - | - | - | - | - | - | - | - | - | 0.2 | - |
| Carbomer (2% aqueous solution) | - | 16.0 | - | 16.0 | - | 15.0 | - | 10.0 | - | 10.0 | - | 10.0 | - | 10.0 |
| KOH | - | 0.5 | - | 0.5 | - | 0.5 | - | 0.3 | - | 0.3 | - | 0.3 | - | 0.3 |
| Citric acid | 0.2 | - | 0.2 | - | 0.2 | - | 0.2 | - | 0.2 | - | 0.2 | - | 0.2 | - |
| Na citrate | 1.0 | - | 1.0 | - | 1.0 | - | 1.0 | - | 1.0 | - | 1.0 | - | 1.0 | - |
| Methylparaben | 0.2 | 0.1 | 0.2 | 0.1 | 0.2 | 0.1 | 0.2 | 0.1 | 0.2 | 0.1 | 0.2 | 0.1 | 0.2 | 0.1 |
| Total | 100 | 100 | 100 | 100 | 100 | 100 | 100 | 100 | 100 | 100 | 100 | 100 | 100 | 100 |
| Viscosity | 45600 | 46900 | 64100 | 47100 | 22700 | 32900 | 25100 | 17700 | 19900 | 20700 | 18700 | 17700 | 17000 | 17700 |
| pH | 6.0 | 6.1 | 5.9 | 6.0 | 6.1 | 6.4 | 6.1 | 6.2 | 6.1 | 6.2 | 6.1 | 6.2 | 6.1 | 6.2 |
| Gelling rate | A | | A | | A | | A | | A | | A | | A | |
| Dripping | A | | A | | A | | A | | A | | A | | A | |
| Film strength | B | | B | | A | | A | | A | | A | | A | |
| Syneresis of gel | A | | A | | A | | A | | A | | A | | A | |
| Feeling of use as peel-off mask | B | | B | | B | | A | | A | | A | | A | |

| | Example | | Example | | Example | | Example | | Example | | Example | | | |
|---|---|---|---|---|---|---|---|---|---|---|---|---|---|---|
| | 8 | | 9 | | 10 | | 11 | | 12 | | 13 | | | |
| | FL | SL | FL | SL | FL | SL | FL | SL | FL | SL | FL | SL | | |
| Purified water | 90.4 | 12.3 | 90.4 | 12.3 | 90.4 | 12.3 | 90.4 | 12.3 | 90.4 | 12.3 | 90.4 | 12.3 | | |
| BG | 5.0 | 11.9 | 5.0 | 21.9 | 5.0 | 31.9 | 5.0 | 41.9 | 5.0 | 51.9 | 5.0 | 61.9 | | |
| Glycerin | - | 60.0 | - | 50.0 | - | 40.0 | - | 30.0 | - | 20.0 | - | 10.0 | | |
| Tamarind gum | 3.0 | - | 3.0 | - | 3.0 | - | 3.0 | - | 3.0 | - | 3.0 | - | | |
| Locust bean gum | 0.2 | - | 0.2 | - | 0.2 | - | 0.2 | - | 0.2 | - | 0.2 | - | | |
| Xanthan gum | - | 0.2 | - | 0.2 | - | 0.2 | - | 0.2 | - | 0.2 | - | 0.2 | | |
| Guar gum | - | - | - | - | - | - | - | - | - | - | - | - | | |
| Tara gum | - | - | - | - | - | - | - | - | - | - | - | - | | |
| Carbomer (2% aqueous solution) | - | 15.0 | - | 15.0 | - | 15.0 | - | 15.0 | - | 15.0 | - | 15.0 | | |
| KOH | - | 0.5 | - | 0.5 | - | 0.5 | - | 0.5 | - | 0.5 | - | 0.5 | | |
| Citric acid | 0.2 | - | 0.2 | - | 0.2 | - | 0.2 | - | 0.2 | - | 0.2 | - | | |
| Na citrate | 1.0 | - | 1.0 | - | 1.0 | - | 1.0 | - | 1.0 | - | 1.0 | - | | |
| Methylparaben | 0.2 | 0.1 | 0.2 | 0.1 | 0.2 | 0.1 | 0.2 | 0.1 | 0.2 | 0.1 | 0.2 | 0.1 | | |
| Total | 100 | 100 | 100 | 100 | 100 | 100 | 100 | 100 | 100 | 100 | 100 | 100 | | |
| Viscosity | 17700 | 65200 | 17700 | 49900 | 17700 | 39200 | 17700 | 35200 | 17700 | 23200 | 17700 | 8590 | | |
| pH | 6.1 | 6.4 | 6.1 | 6.4 | 6.1 | 6.4 | 6.1 | 6.4 | 6.1 | 6.4 | 6.1 | 6.4 | | |
| Gelling rate | B | | B | | B | | A | | A | | A | | | |
| Dripping | A | | A | | A | | A | | B | | B | | | |
| Film strength | B | | B | | B | | B | | B | | B | | | |
| Syneresis of gel | A | | A | | A | | A | | B | | B | | | |
| Feeling of use as peel-off mask | A | | A | | A | | A | | B | | B | | | |

**[Table 2]**

| | Comparative Example | | Comparative Example | | Comparative Example | | Comparative Example | |
|---|---|---|---|---|---|---|---|---|
| | 1 | | 2 | | 3 | | 4 | |
| | FL | SL | FL | SL | FL | SL | FL | SL |
| Purified water | 90.1 | 17.5 | 89.9 | 17.7 | 90.1 | 17.7 | 93.4 | 17.5 |
| BG | 5.0 | 41.9 | 5.0 | 41.9 | 5.0 | 41.9 | 5.0 | 41.9 |
| Glycerin | - | 30.0 | - | 30.0 | - | 30.0 | - | 30.0 |
| Tamarind gum | 3.5 | - | 3.5 | - | 3.5 | - | - | - |
| Locust bean gum | - | - | 0.2 | - | - | - | 0.2 | - |
| Xanthan gum | - | 0.2 | - | - | - | - | - | 0.2 |
| Guar gum | - | - | - | - | - | - | - | - |
| Tara gum | - | - | - | - | - | - | - | - |
| Carbomer (2% aqueous solution) | - | 10.0 | - | 10.0 | - | 10.0 | - | 10.0 |
| KOH | - | 0.3 | - | 0.3 | - | 0.3 | - | 0.3 |
| Citric acid | 0.2 | - | 0.2 | - | 0.2 | - | 0.2 | - |
| Na citrate | 1.0 | - | 1.0 | - | 1.0 | - | 1.0 | - |
| Methylparaben | 0.2 | 0.1 | 0.2 | 0.1 | 0.2 | 0.1 | 0.2 | 0.1 |
| Total | 100 | 100 | 100 | 100 | 100 | 100 | 100 | 100 |
| Viscosity | 9000 | 14000 | 22900 | 26700 | 9000 | 26700 | 7 | 17700 |
| pH | 6.1 | 6.2 | 6.1 | 6.2 | 6.1 | 6.2 | 6.1 | 6.2 |
| Gelling rate | A | | A | | A | | D | |
| Dripping | A | | B | | A | | D | |
| Film strength | B | | B | | B | | D | |
| Syneresis of gel | D | | D | | D | | D | |
| Feeling of use as peel-off mask | C | | C | | C | | D | |

In the Tables, "FL" denotes "First liquid" and "SL" denotes "Second liquid". In Tables above, the numerical values of the raw materials indicate amounts in parts by mass. The following raw materials were used.
BG: 1,3-butylene glycol
Tamarind gum: GLYLOID 6C (DSP Gokyo Food & Chemical Co., Ltd.)
Locust bean gum: GENUGUM RL-200-J (Sansho Co., Ltd.)
Xanthan gum: KELDENT (DSP Gokyo Food & Chemical Co., Ltd.)
Guar gum: GUAPACK PF-20 (Sansho Co., Ltd.)
Tara gum: tara gum MT120 (Mitsubishi-Chemical Foods Corporation)
Carbomer: AQUPEC HV-805EG-300 (CT-1) (2% aqueous solution) (Sumitomo Seika Chemicals Co., Ltd.)
KOH: 10%-by-mass aqueous potassium hydroxide solution
Citric acid: 10%-by-mass aqueous citric acid solution
Na citrate: 10%-by-mass aqueous sodium citrate solution

### [Reference Signs List]

10: aerosol product, 11: outer container, 12: aerosol valve, 13A: first housing, 13B: second housing, 14A: first stem, 14B: second stem, 15A: first inner bag, 15B: second inner bag, 16: propellant filling space, 21: actuator, 22A: first actuator passage, 22B: second actuator passage,
30: aerosol product, 31a: first outer container, 31b: second outer container, 32a: first inner container, 32b: second inner container, 33a: first propellant filling space, 33b: second propellant filling space, 35: actuator passage, 36: actuator, 37: fixation plate

## Claims

1. A gelled aerosol product adapted for cosmetics, comprising: a first container filled with a first liquid; a second container filled with a second liquid; and a propellant, and having a discharge mechanism for discharging the first liquid and the second liquid,
wherein
the first liquid comprises either one of the following (A) and (B), and tamarind gum,
the second liquid comprises the other of the following (A) and (B), and glycerin and a diol having 2 to 6 carbon atoms:
(A) at least one selected from the group consisting of locust bean gum, glucomannan, tara gum, and guar gum; and
(B) xanthan gum, and
the first liquid and the second liquid that are discharged are gelled by mixing.

2. The gelled aerosol product according to claim 1, wherein the (A) is at least one selected from the group consisting of locust bean gum, tara gum, and guar gum.

3. The gelled aerosol product according to claim 1, wherein the (A) is locust bean gum.

4. The gelled aerosol product according to any one of claims 1 to 3, wherein a ratio of the (A) and the (B), [(A):(B)], is 1:3 to 3:1 in terms of mass.

5. The gelled aerosol product according to any one of claims 1 to 4, wherein the diol having 2 to 6 carbon atoms is at least one selected from the group consisting of 1,3-butylene glycol, propylene glycol, isopentyl diol, pentylene glycol, and 1,3-propanediol.

6. The gelled aerosol product according to any one of claims 1 to 5, wherein a mass ratio of the diol having 2 to 6 carbon atoms with respect to the glycerin (diol having 2 to 6 carbon atoms/glycerin) is 0.15 to 7.0.

7. The gelled aerosol product according to any one of claims 1 to 6, wherein a content of tamarind gum in the first liquid is 1.0% by mass to 10.0% by mass.

8. The gelled aerosol product according to any one of claims 1 to 7, comprising an outer container which comprises: a propellant filling space filled with the propellant; the first container; and the second container, and having a discharge mechanism for discharging the first liquid and the second liquid from the first container and the second container, respectively,
wherein the propellant filling space is formed between the first container, the second container and the outer container.

9. The gelled aerosol product according to any one of claims 1 to 7, comprising: a first inner container filled with the first liquid; a second inner container filled with the second liquid; a first outer container in which the first inner container is housed; and a second outer container in which the second inner container is housed, and having a discharge mechanism for discharging the first liquid and the second liquid,
wherein the propellant is filled into each of a space formed between the first inner container and the first outer container and a space formed between the second inner container and the second outer container.

10. A gel forming kit adapted for cosmetics, at least comprising separate containers each filled with a first liquid or a second liquid,
wherein
the first liquid comprises either one of the following (A) and (B), and tamarind gum,
the second liquid comprises the other of the following (A) and (B), and glycerin and a diol having 2 to 6 carbon atoms:
(A) at least one selected from the group consisting of locust bean gum, glucomannan, tara gum, and guar gum; and
(B) xanthan gum, and
the first liquid and the second liquid are gelled by mixing.

11. The gel forming kit according to claim 10, wherein the (A) is at least one selected from the group consisting of locust bean gum, tara gum, and guar gum.

12. The gel forming kit according to claim 10 or 11, wherein a ratio of the (A) and the (B), [(A):(B)], is 1:3 to 3:1 in terms of mass.

13. The gel forming kit according to any one of claims 10 to 12, wherein the diol having 2 to 6 carbon atoms is at least one selected from the group consisting of 1,3-butylene glycol, propylene glycol, isopentyl diol, pentylene glycol, and 1,3-propanediol.

14. The gel forming kit according to any one of claims 10 to 13, wherein a mass ratio of the diol having 2 to 6 carbon atoms with respect to the glycerin (diol having 2 to 6 carbon atoms/glycerin) is 0.15 to 7.0.

15. The gel forming kit according to any one of claims 10 to 14, wherein a content of tamarind gum in the first liquid is 1.0% by mass to 10.0% by mass.

## Patentansprüche

1. Geliertes Aerosolprodukt, das für Kosmetika geeignet ist, umfassend: einen ersten Behälter, der mit einer ersten Flüssigkeit gefüllt ist; einen zweiten Behälter, der mit einer zweiten Flüssigkeit gefüllt ist; und ein Treibmittel, und aufweisend einen Abgabemechanismus zum Abgeben der ersten Flüssigkeit und der zweiten Flüssigkeit,
wobei
die erste Flüssigkeit eines von den folgenden (A) und (B) und Tamarindkernmehl umfasst,
die zweite Flüssigkeit das andere von den folgenden (A) und (B) und Glycerin und ein Diol mit 2 bis 6 Kohlenstoffatomen umfasst:
(A) mindestens eines, das aus der Gruppe bestehend aus Johannisbrotkernmehl, Glucomannan, Tarakernmehl und Guarkernmehl ausgewählt ist; und
(B) Xanthankernmehl, und
die erste Flüssigkeit und die zweite Flüssigkeit, die abgegeben werden, durch Mischen geliert werden.

2. Geliertes Aerosolprodukt nach Anspruch 1, wobei das (A) mindestens eines ist, das aus der Gruppe bestehend aus Johannisbrotkernmehl, Tarakernmehl und Guarkernmehl ausgewählt ist.

3. Geliertes Aerosolprodukt nach Anspruch 1, wobei das (A) Johannisbrotkernmehl ist.

4. Geliertes Aerosolprodukt nach einem der Ansprüche 1 bis 3, wobei ein Verhältnis des (A) und des (B), [(A): (B)], 1:3 bis 3:1 in Bezug auf die Masse beträgt.

5. Geliertes Aerosolprodukt nach einem der Ansprüche 1 bis 4, wobei das Diol mit 2 bis 6 Kohlenstoffatomen mindestens eines ist, das aus der Gruppe bestehend aus 1,3-Butylenglykol, Propylenglykol, Isopentyldiol, Pentylenglykol und 1,3-Propandiol ausgewählt ist.

6. Geliertes Aerosolprodukt nach einem der Ansprüche 1 bis 5, wobei ein Massenverhältnis des Diols mit 2 bis 6 Kohlenstoffatomen in Bezug auf das Glycerin (Diol mit 2 bis 6 Kohlenstoffatomen/Glycerin) 0,15 bis 7,0 beträgt.

7. Geliertes Aerosolprodukt nach einem der Ansprüche 1 bis 6, wobei ein Gehalt von Tamarindkernmehl in der ersten Flüssigkeit 1,0 Massen-% bis 10,0 Massen-% beträgt.

8. Geliertes Aerosolprodukt nach einem der Ansprüche 1 bis 7, umfassend einen Außenbehälter, der umfasst: einen Treibmittelfüllraum, der mit dem Treibmittel gefüllt ist; den ersten Behälter und den zweiten Behälter, und aufweisend einen Abgabemechanismus zum Abgeben der ersten Flüssigkeit und der zweiten Flüssigkeit aus dem ersten Behälter bzw. dem zweiten Behälter,
wobei der Treibmittelfüllraum zwischen dem ersten Behälter, dem zweiten Behälter und dem Außenbehälter geformt ist.

9. Geliertes Aerosolprodukt nach einem der Ansprüche 1 bis 7, umfassend: einen ersten Innenbehälter, der mit der ersten Flüssigkeit gefüllt ist; einen zweiten Innenbehälter, der mit der zweiten Flüssigkeit gefüllt ist; einen ersten Außenbehälter, in dem der erste Innenbehälter untergebracht ist; und einen zweiten Außenbehälter, in dem der zweite Innenbehälter untergebracht ist, und aufweisend einen Abgabemechanismus zum Abgeben der ersten Flüssigkeit und der zweiten Flüssigkeit,
wobei das Treibmittel in jeden von einem Raum, der zwischen dem ersten Innenbehälter und dem ersten Außenbehälter geformt ist, und einem Raum, der zwischen dem zweiten Innenbehälter und dem zweiten Außenbehälter geformt ist, gefüllt wird.

10. Gelbildungskit, das für Kosmetika geeignet ist, mindestens umfassend separate Behälter, die jeweils mit einer ersten Flüssigkeit oder einer zweiten Flüssigkeit gefüllt sind,
wobei
die erste Flüssigkeit eines von den folgenden (A) und (B) und Tamarindkernmehl umfasst,
die zweite Flüssigkeit das andere von den folgenden (A) und (B) und Glycerin und ein Diol mit 2 bis 6 Kohlenstoffatomen umfasst:
(A) mindestens eines, das aus der Gruppe bestehend aus Johannisbrotkernmehl, Glucomannan, Tarakernmehl und Guarkernmehl ausgewählt ist; und
(B) Xanthankernmehl, und
die erste Flüssigkeit und die zweite Flüssigkeit durch Mischen geliert werden.

11. Gelbildungskit nach Anspruch 10, wobei das (A) mindestens eines ist, das aus der Gruppe bestehend aus Johannisbrotkernmehl, Tarakernmehl und Guarkernmehl ausgewählt ist.

12. Gelbildungskit nach Anspruch 10 oder 11, wobei ein Verhältnis des (A) und des (B), [(A):(B)], 1:3 bis 3:1 in Bezug auf die Masse beträgt.

13. Gelbildungskit nach einem der Ansprüche 10 bis 12, wobei das Diol mit 2 bis 6 Kohlenstoffatomen mindestens eines ist, das aus der Gruppe bestehend aus 1,3-Butylenglykol, Propylenglykol, Isopentyldiol, Pentylenglykol und 1,3-Propandiol ausgewählt ist.

14. Gelbildungskit nach einem der Ansprüche 10 bis 13, wobei ein Massenverhältnis des Diols mit 2 bis 6 Kohlenstoffatomen in Bezug auf das Glycerin (Diol mit 2 bis 6 Kohlenstoffatomen/Glycerin) 0,15 bis 7,0 beträgt.

15. Gelbildungskit nach einem der Ansprüche 10 bis 14, wobei ein Gehalt von Tamarindkernmehl in der ersten Flüssigkeit 1,0 Massen-% bis 10,0 Massen-% beträgt.

## Revendications

1. Produit en aérosol gélifié adapté pour des cosmétiques, comprenant : un premier récipient rempli d'un premier liquide ; un deuxième récipient rempli d'un deuxième liquide ; et un propulseur, et ayant un mécanisme de décharge pour décharger le premier liquide et le deuxième liquide, dans lequel
le premier liquide comprend l'un ou l'autre parmi les (A) et (B) qui suivent, et de la gomme de tamarin,
le deuxième liquide comprend l'un ou l'autre parmi les (A) et (B) qui suivent, et de la glycérine et un diol ayant 2 à 6 atomes de carbone :
(A) au moins l'un choisi dans le groupe constitué par la gomme de caroube, le glucomannane, la gomme tara, et la gomme de guar ; et
(B) la gomme xanthane, et
le premier et le deuxième liquide qui sont déchargés sont gélifiés par mélange.

2. Produit en aérosol gélifié selon la revendication 1, dans lequel (A) est au moins l'une choisie dans le groupe constitué par la gomme de caroube, la gomme tara, et la gomme de guar.

3. Produit en aérosol gélifié selon la revendication 1, dans lequel (A) est la gomme de caroube.

4. Produit en aérosol gélifié selon l'une quelconque des revendications 1 à 3, dans lequel le rapport de (A) à (B), [(A)/(B)], est de 1/3 en 3/1 en masse.

5. Produit en aérosol gélifié selon l'une quelconque des revendications 1 à 4, dans lequel le diol ayant 2 à 6 atomes de carbone est au moins l'un choisi dans le groupe constitué par le 1,3-butylèneglycol, le propylèneglycol, l'isopentyldiol, le pentylèneglycol, et le 1,3-propanediol.

6. Produit en aérosol gélifié selon l'une quelconque des revendications 1 à 5, dans lequel le rapport en masse du diol ayant 2 à 6 atomes de carbone à la glycérine (diol ayant 2 à 6 atomes de carbone/glycérine) est de 0,15 à 7,0.

7. Produit en aérosol gélifié selon l'une quelconque des revendications 1 à 6, dans lequel la teneur en gomme de tamarin dans le premier liquide est de 1,0 % en masse à 10,0 % en masse.

8. Produit en aérosol gélifié selon l'une quelconque des revendications 1 à 7, comprenant un récipient extérieur qui comprend : un espace de remplissage par un propulseur rempli du propulseur ; le premier récipient ; et le deuxième récipient, et ayant un mécanisme de décharge pour décharger le premier liquide et le deuxième liquide respectivement hors du premier récipient et hors du deuxième récipient,
dans lequel l'espace de remplissage par un propulseur est formé entre le premier récipient, le deuxième récipient et le récipient extérieur.

9. Produit en aérosol gélifié selon l'une quelconque des revendications 1 à 7, comprenant : un premier récipient intérieur rempli du premier liquide ; un deuxième récipient intérieur rempli du deuxième liquide ; un premier récipient extérieur dans lequel est logé le premier récipient intérieur ; et un deuxième récipient extérieur dans lequel est logé le deuxième récipient intérieur, et ayant un mécanisme de décharge pour décharger le premier liquide et le deuxième liquide,
dans lequel le propulseur remplit chacun parmi un espace formé entre le premier récipient intérieur et le premier récipient extérieur, et un espace formé entre le deuxième récipient intérieur et le deuxième récipient extérieur.

10. Kit de formation de gel adapté pour des cosmétiques, comprenant au moins des récipients séparés remplis chacun par un premier liquide ou un deuxième liquide, dans lequel
le premier liquide comprend l'un ou l'autre parmi les (A) et (B) qui suivent, et de la gomme de tamarin,
le deuxième liquide comprend l'un ou l'autre parmi les (A) et (B) qui suivent, et de la glycérine et un diol ayant 2 à 6 atomes de carbone :
(A) au moins l'un choisi dans le groupe constitué par la gomme de caroube, le glucomannane, la gomme tara, et la gomme de guar ; et
(B) la gomme xanthane, et
le premier et le deuxième liquide sont gélifiés par mélange.

11. Kit de formation de gel selon la revendication 10, dans lequel (A) est au moins l'une choisie dans le groupe constitué par la gomme de caroube, la gomme tara, et la gomme de guar.

12. Kit de formation de gel selon la revendication 10 ou 11, dans lequel le rapport de (A) à (B), [(A)/(B)], est de 1/3 en 3/1 en masse.

13. Kit de formation de gel selon l'une quelconque des revendications 10 à 12, dans lequel le diol ayant 2 à 6 atomes de carbone est au moins l'un choisi dans le groupe constitué par le 1,3-butylèneglycol, le propylèneglycol, l'isopentyldiol, le pentylèneglycol, et le 1,3-propanediol.

14. Kit de formation de gel selon l'une quelconque des revendications 10 à 13, dans lequel le rapport en masse du diol ayant 2 à 6 atomes de carbone à la glycérine (diol ayant 2 à 6 atomes de carbone/glycérine) est de 0,15 à 7,0.

15. Kit de formation de gel selon l'une quelconque des revendications 10 à 14, dans lequel la teneur en gomme de tamarin dans le premier liquide est de 1,0 % en masse à 10,0 % en masse.
